# DEMANDE DE BREVET EUROPEEN

(11) **EP 4 483 862 A1**
(43) Date de publication de la demande: **01.01.2025**
(21) Numéro de dépôt: 23306056.5
(22) Date de dépôt: 28.06.2023
(51) Int. Cl.: A61K 8/60, A61K 8/9789, A61Q 17/00, A61Q 19/00

(54) **EXTRAIT ALCOOLIQUE DE PARTIES AÉRIENNES DE PLANTAGO LANCEOLATA, SON PROCÉDÉ D'OBTENTION, ET COMPOSITION COSMÉTIQUE LE CONTENANT**

(71) Demandeur: Chanel Parfums Beauté, 92200 Neuilly-sur-Seine (FR)
(72) Inventeur: LEGANGNEUX, DAVID, 93694 PANTIN CEDEX (FR); COCANDEAU, VINCENT, 93694 PANTIN CEDEX (FR)
(74) Mandataire: Plasseraud IP

(57) **Abrégé**

L'invention concerne un extrait de parties aériennes de *Plantago lanceolata* son procédé d'obtention, et composition cosmétique le contenant, ainsi que différentes utilisations cosmétiques.

## Description

L'invention concerne un extrait des parties aériennes de *Plantago lanceolata,* son procédé d'obtention, une composition cosmétique le contenant, ainsi que différentes utilisations cosmétiques.

La peau est principalement constituée de trois couches, à savoir, en partant de la plus superficielle, l'épiderme, le derme et l'hypoderme. L'épiderme contribue largement à assurer la protection de la peau et à en maintenir son bon fonctionnement. L'épiderme joue à la fois un rôle de barrière hydrique et physique.

L'épiderme elle-même est également constituée de quatre à cinq couches et chacune de ces couches correspond un état de différenciation des kératinocytes (le type cellulaire majoritaire de l'épiderme). Ces couches sont, en partant de la plus superficielle, la couche cornée, le stratum lucidum (présent que dans les épidermes épais), la couche granuleuse, la couche épineuse et la couche basale. Ainsi, la couche basale, la plus profonde, est le compartiment germinatif ou prolifératif qui donnent naissance aux kératinocytes des couches plus superficielles. Les kératinocytes possèdent de solides structures d'adhésion telles que les desmosomes et les jonctions serrées qui leur permet de s'ancrer aux autres kératinocytes.

La fonction primaire de l'épiderme est de produire la couche cornée qui forme une couche protectrice semi-perméable en empêchant la perte en eau, en maintenant une hydratation satisfaisante de la peau et en évitant une hyperhydratation. Ainsi, l'épiderme jour le rôle de barrière hydrique.

Etant donné que la peau est la première barrière de protection de l'organisme vis-à-vis de l'environnement, elle subit donc de nombreuses agressions extérieures qui peuvent conduire à des réactions cutanées d'inconfort voire, en cas de réactions très intenses ou plus graves, à des phénomènes d'irritation et/ou d'inflammation cutanées.

Les réactions cutanées d'inconfort, voire alternativement des réactions d'irritation et/ou d'inflammation de la peau peuvent notamment être induites par le contact avec des produits chimiques tels que les nettoyants, ou provenir d'actions mécaniques tels que le rasage, le gommage, le peeling, l'épilation.

Il existe toujours un besoin pour de nouveaux agents apaisants dans le domaine cosmétique. Il existe également un besoin pour de nouveaux agents, aptes à traiter et/ou diminuer les réactions d'irritation et/ou d'inflammation cutanées dans le domaine dermatologique. Il existe également un besoin pour prévenir une diminution de et/ou renforcer la fonction barrière cutanée pour restaurer physiologiquement un état d'hydratation convenable à la couche cornée.

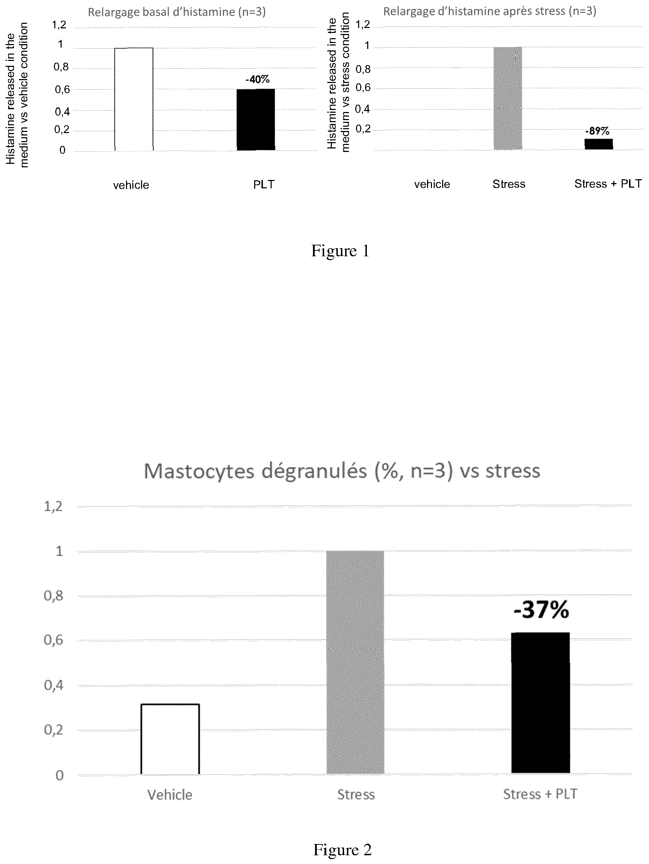

Or, la Demanderesse a maintenant trouvé qu'un extrait alcoolique de parties aériennes de *Plantago lanceolata,* obtenu par un procédé particulier, présente, par le biais de stimulation ou d'inhibition de mécanismes physiologiques, des activités intéressantes vis-à-vis du renforcement de la fonction barrière cutanée et la réduction de l'inflammation. En effet, comme démontré en exemples, l'extrait alcoolique de parties aériennes de *Plantago lanceolata,* selon l'invention présente des propriétés cosmétiques intéressantes : il permet une stimulation significative des voies biologiques en lien avec la fonction barrière cutanée et notamment des marqueurs de la différenciation de l'épiderme tels que les SPRR, CNFN, LCE3D, SBSN, FLG1 ainsi que des marqueurs plus spécifiques des desmosomes tels que CDSN, DSG1 ou des jonctions serrées comme CLDN7 ou bien l'AQP9 qui est également un gène impliqué dans le maintien d'une bonne hydratation cutanée.

Par sa capacité à stimuler des cibles impliquées dans la différenciation de l'épiderme et la rétention d'eau dans la peau, l'extrait selon l'invention montre des propriétés cosmétiques de renforcement de la fonction barrière et d'hydratation.

D'autre part, l'extrait présente des propriétés cosmétiques apaisantes car il inhibe les voies biologiques de la neuro-inflammation et celle des interférons, des cytokines immunomodulatrices connues pour leur rôle régulateur dans l'inflammation cutanée.

Le document FR 2 850 572 propose l'utilisation du verbascoside comme agent de stimulation de la synthèse des protéines de choc thermique et l'inhibition de la collagenase et de l'élastase par les cellules de la peau. Toutefois, ce document ne décrit pas un extrait alcoolique comprenant, en plus du verbascoside, d'autres composés, tels que l'aucubin et le catalpol.

En effet, dans ce document, le verbascoside seul est décrit comme jouant un rôle dans la lutte contre le vieillissement de la peau et la pollution environnementale par sa capacité de stimuler la synthèse de protéines de choc thermique, et plus particulièrement des HSP70. Selon ce document, la synthèse des HSP constitutive évolue avec l'âge et, de manière générale, tend vers une diminution dans les tissus. L'accroissement de l'expression HSP70 par le verbascoside peut ainsi être considérée, selon ce document, comme un moyen de l'accroissement de la lutte contre la pollution environnementale.

Toutefois, un tel extrait ne permet pas d'agir sur les gènes impliqués dans les voies liées à l'inflammation dans les kératinocytes humains normaux ou au renforcement de la fonction barrière/hydratation.

L'invention concerne donc, selon un premier aspect, un extrait alcoolique de parties aériennes de *Plantago lanceolata,* comprenant au moins de l'aucubin, du catalpol et du verbascoside.

L'invention a également pour objet, selon un second aspect, un procédé d'extraction de parties aériennes de *Plantago lanceolata,* comprenant au moins les étapes suivantes :
a) le broyage des parties aériennes de Plantago lanceolata,
b) au moins une, de préférence au moins deux extractions desdites parties aériennes broyées en présence d'un premier solvant mono-alcoolique, chaque extraction étant suivie d'une étape de filtration ;
c) le mélange des filtrats liquides issus des extractions de l'étape b) ; filtration du mélange obtenu et, optionnellement, décoloration du filtrat obtenu par adsoption sur charbon actif ;
d) la concentration du mélange obtenu à l'étape c), pour obtenir un extrait ayant une teneur en matière sèche comprise entre 8% et 13%, le reste de l'extrait étant composé du premier solvant mono-alcoolique;
e) l'ajout d'un deuxième solvant alcoolique à l'extrait concentré obtenu à l'étape d), ledit deuxième solvant alcoolique étant différent du premier solvant mono-alcoolique utilisé à l'étape b), suivi d'une élimination du premier solvant mono-alcoolique jusqu'à une concentration finale inférieure à 0.5% ; et optionnellement la filtration de l'extrait obtenu.

L'invention se rapporte également à un extrait alcoolique de parties aériennes de *Plantago lanceolata* susceptible d'être obtenu par un tel procédé.

L'invention se rapporte aussi à une utilisation cosmétique d'un extrait alcoolique de parties aériennes de *Plantago lanceolata* obtenu par un tel procédé, pour le renforcement de la fonction barrière et d'hydratation ou comme agent apaisant.

L'invention se rapporte en outre aussi à une composition cosmétique comprenant, dans un véhicule cosmétiquement acceptable, un extrait alcoolique de parties aériennes de *Plantago lanceolata,* selon le premier aspect. Par véhicule cosmétiquement acceptable, on entend un milieu compatible avec la peau, les muqueuses et les phanères. De préférence, la composition cosmétique selon l'invention est adaptée à une application par voie topique.

### Figures

La Figure 1 illustre le dosage de l'histamine relarguée dans le milieu de culture d'explants traités ou non avec l'extrait selon l'invention en conditions basales (à gauche) ou stressés (à droite).
La Figure 2 illustre le comptage de mastocytes ayant dégranulé dans des explants traités ou non avec l'extrait selon l'invention en conditions stressés

La matière première mise en oeuvre est constituée de parties aériennes de *Plantago lanceolata.*

Plantain lancéolé *(Plantago lanceolata)* appelée aussi petit plantain, herbe aux cinq coutures, herbe à 5 côtes, herbe à la couture est une plante herbacée vivace, atteignant 10 à 60 cm de haut, présentant des rosettes denses de feuilles basilaires et une longue hampe florale. Les feuilles sont vertes, couvertes de poils, ovales et lancéolées, mesurant de 10 à 25 cm de long, et présentant 5 à 9 nervures parallèles convergentes. La hampe florale de 60 cm de haut, est surmontée d'un épi cylindrique de minuscules fleurs vertes très tendres. La floraison a lieu d'avril à octobre.

Elle est originaire d'Europe, d'Afrique du Nord, d'Asie tempérée et tropicale et a été introduite partout dans le monde.

C'est une espèce que pousse entre 0 et 2000 mètres dans un emplacement ensoleillé ou mi-ombragé et dans un sol riche, plutôt acide et bien drainé. Elle pousse généralement dans les pâturages, les pelouses et les champs.

Les parties aériennes de *Plantago lanceolata* utilisées selon l'invention sont typiquement choisies parmi les fleurs, les feuilles, les tiges et leurs mélanges. De préférence, les parties aériennes utilisées sont un mélange de fleurs, feuilles et tiges de *Plantago lanceolata.* De préférence, ces parties aériennes sont préalablement séchées, puis broyées ou réduites en morceaux de manière usuelle, pour, de préférence, se présenter sous la forme d'une poudre de taille inférieure à 2 cm.

L'extrait sec de parties aériennes de *Plantago lanceolata* objet de la présente invention est composé de plusieurs familles de métabolites primaires et secondaires d'intérêt.

L'extrait alcoolique des parties aériennes de *Plantago lanceolata* objet de la présente invention comprend notamment au moins de l'aucubin, du catalpol et du verbascoside.

En particulier, l'extrait alcoolique de *Plantago lanceolata* de l'invention comprend
- 10 à 50% en poids de sucres et de sorbitol, de préférence 20 à 40% en poids,
- 1 à 10% en poids d'aucubin, de préférence 6 à 9% en poids,
- 20 à 40% en poids de catalpol, de préférence 25 à 35% en poids, et
- 5 à 15% en poids de verbascoside, de préférence 10 à 12% en poids,
les pourcentages étant exprimés en poids, par rapport au poids total de l'extrait sec.

En effet, une quantité importante d'iridoïdes est présente dans l'extrait comprise entre 20 et 70% en poids, de préférence entre 30 et 50% en poids sec par rapport au poids total de l'extrait sec.

Ces iridoïdes correspondent notamment à deux composés de formules brutes C₁₅H₂₂O₉ et C₁₅H₂₂O₁₀ respectivement attribuées à l'aucubin et au catalpol.

L'aucubin (I), le catalpol (II) sont des composés ayant les structures suivantes :

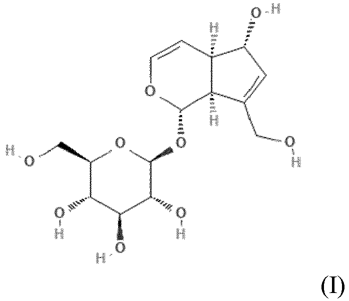

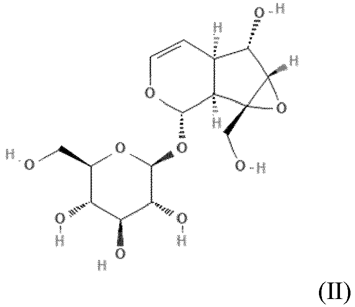

L'extrait comprend également le verbascoside, présent entre 5 à 15% en poids, de préférence entre 10 à 12% en poids par rapport au poids total de l'extrait sec.

Le verbascoside (III) est un composé ayant la structure suivante :

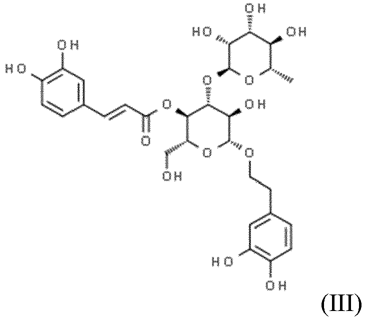

Il est donc du mérite de la demanderesse d'avoir réussi à préparer un extrait de parties aériennes de *Plantago lanceolata* comprenant notamment de l'aucubin, du catalpol et du verbascoside, ce qui en fait sa spécificité.

L'extrait des parties aériennes de *Plantago lanceolata* contient diverses familles de métabolites primaires et secondaires.

Parmi les métabolites primaires, la présence de sucres et de polyols représente entre 10 à 50% en poids de sucres et de sorbitol, de préférence 20 à 40% en poids par rapport au poids total de l'extrait sec. Plus particulièrement, l'extrait sec est principalement composé de sorbitol, de glucose et de sucrose. Avantageusement, les sucres comprennent du glucose et du sucrose, de préférence 1 à 7% en poids de glucose et 1 à 7% en poids de sucrose, les pourcentages étant exprimés en poids, par rapport au poids total de l'extrait.

De préférence, l'extrait selon l'invention comprend entre 20 et 30% en poids de sorbitol, de préférence entre 22 et 26% en poids de sorbitol par rapport au poids total de l'extrait sec. De préférence, l'extrait selon l'invention comprend entre 1 et 5% en poids de glucose. De préférence, l'extrait selon l'invention comprend entre 1 et 5% en poids de sucrose.

L'extrait alcoolique de parties aériennes *de Plantago lanceolata* de l'invention peut notamment être obtenu au moyen d'un procédé d'extraction des parties aériennes de *Plantago lanceolata* comprenant les étapes suivantes :
a) le broyage des parties aériennes de *Plantago lanceolata,*
b) au moins une, de préférence au moins deux extractions desdites parties aériennes broyées en présence d'un premier solvant mono-alcoolique, chaque extraction étant suivie d'une étape de filtration ;
c) le mélange des filtrats liquides issus des extractions de l'étape b) ; filtration du mélange obtenu et, optionnellement, décoloration du filtrat obtenu par adsorption sur charbon actif ;
d) la concentration du mélange obtenu à l'étape c), pour obtenir un extrait ayant une teneur en matière sèche comprise entre 8% et 13%, le reste de l'extrait étant composé du premier solvant mono-alcoolique ;
e) l'ajout d'un deuxième solvant alcoolique à l'extrait concentré obtenu à l'étape d), ledit deuxième solvant alcoolique étant différent du premier solvant mono-alcoolique utilisé à l'étape b), suivi d'une élimination du premier solvant mono-alcoolique jusqu'à une concentration finale inférieure à 0,5% ; et optionnellement la filtration de l'extrait obtenu.

De préférence, les parties aériennes *de Plantago lanceolata* mises en oeuvre à l'étape a) sont séchées et sont typiquement choisies parmi les fleurs, les feuilles, les tiges et leurs mélanges.

Elles sont notamment broyées ou réduites en morceaux, par exemple à une finesse inférieure à 2 cm, par tout moyen connu de l'homme du métier.

Dans l'étape b), les parties aériennes de *Plantago lanceolata* broyées sont soumises à au moins une, de préférence au moins deux extractions par un ou plusieurs solvants monoalcooliques, par exemple choisis parmi les monoalcools comprenant de 1 à 4 atomes de carbone (en C₁-C₄), tels que par exemple le méthanol, l'éthanol ou l'isopropanol.

De préférence, le premier solvant mono-alcoolique est un monoalcool comprenant de 2 à 4 atomes de carbone, plus préférentiellement l'éthanol.

De préférence, le premier solvant mono-alcoolique mis en œuvre en mélange à au moins 90% avec de l'eau, de préférence au moins 95% avec de l'eau.

De préférence, l'éthanol et est mis en oeuvre en mélange à au moins 90% avec de l'eau, de préférence au moins 95% avec de l'eau.

En particulier, l'éthanol est mis en oeuvre en mélange à 96% avec de l'eau.

L'extraction est généralement réalisée en immergeant et en agitant doucement les parties aériennes broyées de*Plantago lanceolata,* de préférence préalablement séchées, dans un ou plusieurs des solvants alcooliques cités ci-dessus, chacune pendant une durée comprise entre 1 et 6 heures, de préférence entre 1 et 3 heures, à une température comprise entre 40°C et 80°C, de préférence entre 50 et 70°C, de préférence encore entre 55 et 65°C.

De préférence, les parties aériennes de *Plantago lanceolata* sont extraites dans le premier solvant mono-alcoolique avec un rapport compris entre 0,5/10 et 2/10, de préférence compris entre 0,8/10 et 1,2/10, de préférence de 1/10 en masse de parties aériennes par rapport à la masse du premier solvant mono-alcoolique.

L'étape d'extraction b) est suivie d'une filtration, par exemple sur un tamis de 50µm, pour éliminer les résidus végétaux.

L'extrait dépourvu des résidus végétaux peut ensuite être laissé à décanter pendant au moins 6h. La décantation peut par exemple se faire en ampoule. Selon un mode préféré de réalisation, l'étape de décantation est réalisée pendant au moins 8h, de préférence au moins 12h, et plus préférentiellement pendant 6 à 18h. La décantation est conduite à une température comprise entre 0 et 25°C, de préférence entre 1 et 10°C, plus préférentiellement entre 4 et 5°C.

Selon un mode préféré de réalisation, l'étape b) met en oeuvre au moins deux extractions. Dans ce cas, chaque extraction étant suivie d'une étape de filtration.

De préférence, une première extraction est réalisée pendant une durée de 2h à une température de 60°C et une deuxième extraction est également réalisée pendant une durée de 2h à une température de 60°C.

A l'issue de la ou des étapes d'extraction b), les filtrats liquides sont récupérés et mélangés.

Le filtrat ou le mélange de filtrats obtenu à l'issue de l'étape c) est alors à nouveau filtré afin d'éliminer les substances insolubles. De préférence, la filtration de l'extrait obtenu en c) est effectuée sur un tamis ou une membrane de filtration de 4 µm. On obtient ainsi un filtrat liquide. Ce filtrat liquide est une fraction alcoolique.

De préférence, entre les étapes d) et e), une étape de décoloration du filtrat obtenu en c) est ajoutée. La décoloration peut se faire par adsorption des pigments présents dans le filtrat sur charbon actif sous agitation pendant une durée comprise entre 1 et 6 heures, de préférence entre 2 et 4 heures, à une température ambiante. De préférence, le charbon actif représente 30% en masse par rapport à la matière sèche d'extrait. Cette étape de décoloration peut être suivie d'une étape de filtration du filtrat décoloré obtenu, sur un tamis ou une membrane de filtration de 4 µm.

A l'étape d), le mélange obtenu à l'étape c) est concentré pour obtenir un extrait ayant une teneur en matière sèche comprise entre 8% et 13%, le reste de l'extrait étant composé du premier solvant mono-alcoolique. De préférence, l'extrait concentré obtenu à l'étape d) a une teneur en matière sèche comprise entre 10% et 12%.

De préférence, la concentration de l'étape d) se fait par évaporation, en particulier par évaporation partielle sous pression réduite, par exemple au moyen d'un évaporateur rotatif ou d'un évaporateur à film râclé.

A l'étape e), un deuxième solvant alcoolique est ensuite ajouté à l'extrait concentré obtenu à l'étape d) pour diluer l'extrait jusqu'ayant une teneur en matière sèche comprise entre 8 et 12%, de préférence entre 9 et 11%, ledit deuxième solvant alcoolique étant différent du premier solvant mono-alcoolique utilisé à l'étape b), suivie d'une élimination du premier solvant mono-alcoolique jusqu'à une concentration finale inférieure à 0,5%.

Le deuxième solvant alcoolique de l'étape e) peut être choisi parmi les monoalcools en C₁-C₄, tels que par exemple le méthanol, l'éthanol, le propanol, l'isopropanol, le butanol ou l'isobutanol ; les polyols, en particulier les diols, tels que par exemple le propylène glycol, le 1,3-propanediol ou le dipropylène glycol, ou les triols tels que la glycérine.

De préférence, le deuxième solvant alcoolique de l'étape e) est un polyol comprenant de 1 à 4 atomes de carbone, plus préférentiellement le 1,3-propanediol.

De manière encore plus préférée, le premier solvant mono-alcoolique de l'étape b) est un monoalcool comprenant de 2 à 4 atomes de carbone, plus préférentiellement l'éthanol, et le deuxième solvant alcoolique de l'étape e) est un polyol comprenant de 1 à 4 atomes de carbone, plus préférentiellement le 1,3-propanediol.

L'étape e) prévoit également une nouvelle étape d'élimination du premier solvant mono-alcoolique utilisé dans l'étape a) après l'étape de dilution dans de deuxième solvant alcoolique de manière à ce que ce premier soit présent dans une concentration inférieure à 5% dans l'extrait, de préférence dans une concentration inférieure à 1% dans l'extrait, de préférence dans une concentration inférieure à 0,5% dans l'extrait. L'élimination du premier solvant mono-alcoolique de l'étape e) se fait par évaporation, en particulier par évaporation totale sous pression réduite, par exemple au moyen d'un évaporateur à film râclé. Avantageusement, cette étape est mise en oeuvre à l'aide d'un évaporateur à film raclé.

L'extrait est finalement filtré sur un tamis ou une membrane de filtration, de préférence de 4 µm.

L'ordre des étapes de l'élimination du premier solvant mono-alcoolique et d'ajout du deuxième solvant alcoolique est indifférent. En particulier, le premier solvant mono-alcoolique présent dans le filtrat liquide peut être éliminé, puis le reste de filtrat est dilué dans un deuxième solvant alcoolique. Alternativement, le filtrat liquide est d'abord dilué dans un deuxième solvant alcoolique, et le premier solvant mono-alcoolique est ensuite éliminé. Encore alternativement, la dilution dans le deuxième solvant alcoolique et l'élimination du premier solvant mono-alcoolique peut être simultanée.

Préférentiellement, l'extrait alcoolique de parties aériennes de *Plantago lanceolata,* selon l'invention est susceptible d'être obtenu par un procédé comprenant les étapes suivantes :
a) le broyage des parties aériennes de *Plantago lanceolata,*
b) au moins une, de préférence au moins deux extractions desdites parties aériennes broyées en présence d'un premier solvant mono-alcoolique, chaque extraction étant suivie d'une étape de filtration ;
c) le mélange des filtrats liquides issus des extractions de l'étape b) ; et filtration du mélange obtenu et, optionnellement, décoloration du filtrat obtenu par adoption sur charbon actif ;
d) la concentration du mélange obtenu à l'étape c), pour obtenir un extrait ayant une teneur en matière sèche comprise entre 8% et 13%, le reste de l'extrait étant composé du premier solvant mono-alcoolique ;
e) l'ajout d'un deuxième solvant alcoolique à l'extrait concentré obtenu à l'étape d), ledit deuxième solvant alcoolique étant différent du premier solvant mono-alcoolique utilisé à l'étape b), suivi d'une élimination du premier solvant mono-alcoolique jusqu'à une concentration finale inférieure à 0.5% ; et optionnellement la filtration de l'extrait obtenu.

Avantageusement, parties aériennes de *Plantago lanceolata* mises en oeuvre à l'étape a) sont séchées.

Avantageusement, l'extrait mis en oeuvre selon l'invention est de couleur orange à ambrée.

Également, ledit extrait se présente sous une forme suffisamment concentrée pour pouvoir être utilisé sans entraîner les problèmes de formulation habituellement rencontrés aux concentrations nécessaires pour obtenir une activité dans les compositions cosmétiques ou dermatologiques sous forme d'émulsion, et sans présenter une couleur foncée, contrairement aux extraits végétaux obtenus par des procédés usuels, lorsqu'ils sont sous forme concentrée.

Dans le cas d'une émulsion, il peut s'agir d'une émulsion eau dans huile ou huile dans eau.

En particulier, après évaporation de l'éthanol, l'extrait de parties aériennes de *Plantago lanceolata,* comprend 5 à 15% en poids d'extrait sec de *Plantago lanceolata* et le reste de 1,3-propanediol, et de préférence, environ 10% en poids d'extrait sec de *Plantago lanceolata* et 90% en poids de 1,3-propanediol.

De ce fait, l'extrait selon l'invention peut être utilisé directement pour la préparation d'une composition cosmétique.

Selon un autre aspect, l'invention concerne l'utilisation cosmétique d'un extrait alcoolique de parties aériennes de *Plantago lanceolata,* selon l'invention, comme actif apaisant.

Selon un autre aspect, l'invention concerne l'utilisation cosmétique d'un extrait alcoolique de parties aériennes de *Plantago lanceolata,* selon l'invention, pour améliorer l'hydratation et/ou améliorer la fonction barrière.

Selon encore un autre aspect, l'invention concerne l'utilisation cosmétique d'un extrait alcoolique de parties aériennes de *Plantago lanceolata* selon l'invention, pour le renforcement de la fonction barrière et d'hydratation ou comme agent apaisant.

En effet, de manière avantageuse, la Demanderesse a trouvé que l'extrait alcoolique de parties aériennes de *Plantago lanceolata* selon l'invention possédait plusieurs activités d'intérêt vis-à-vis de mécanismes physiologiques préventifs ou réparateurs liés à la fonction barrière cutanée et l'hydratation ou bien l'inflammation.

La Demanderesse a notamment trouvé, de manière avantageuse, que l'extrait alcoolique de parties aériennes de *Plantago lanceolata* selon l'invention permettait une augmentation de l'expression des marqueurs de la différenciation de l'épiderme tels que les SPRR, CNFN, LCE3D, SBSN, FLG1 ainsi que des marqueurs plus spécifiques des desmosomes tels que CDSN, DSG1 ou des jonctions serrées comme CLDN72. L'AQP9 est également un gène impliqué dans le maintien d'une bonne hydratation cutanée3. En effet, par sa capacité à stimuler des cibles impliquées dans la différenciation de l'épiderme et la rétention d'eau dans la peau, l'extrait selon l'invention montre des propriétés cosmétiques de renforcement de la fonction barrière et d'hydratation.

D'autre part, l'extrait joue un rôle dans la régulation de l'inflammation cutanée et, par conséquent est reconnue comme ayant des propriétés cosmétiques apaisantes.

Selon encore un autre aspect, l'invention concerne une composition cosmétique comprenant, dans un véhicule cosmétiquement acceptable, un extrait alcoolique de parties aériennes de *Plantago lanceolata* selon les aspects précédents. De préférence, la composition cosmétique est adaptée à une application par voie topique.

De préférence, ledit extrait est présent dans la composition cosmétique ou dermatologique à raison de 0,001 à 10% en poids, par rapport au poids total de la composition, en particulier à raison de 0,01 à 10% en poids, de préférence de 0,1 à 5% en poids. Ladite composition cosmétique peut notamment, être adaptée à une application par voie topique.

La composition cosmétique selon l'invention peut se présenter sous la forme d'une composition de soin et/ou de maquillage des matières kératiniques, de préférence du visage.

En particulier, la composition de l'invention peut se présenter sous la forme d'une composition de soin anti-âge de la peau, en particulier du visage.

Selon un autre mode de réalisation, une composition de l'invention peut se présenter sous la forme d'une composition de maquillage du visage, en particulier sous la forme d'un fond de teint, d'une base de teint ou d'une crème teintée.

La composition cosmétique selon l'invention peut comprendre également un solvant choisi en fonction des différents ingrédients et de la forme d'administration.

A titre d'exemples, on peut citer l'eau (de préférence de l'eau déminéralisée ou des eaux florales), un alcool tel que l'éthanol.

Ladite composition cosmétique peut également comprendre, en outre de l'extrait selon l'invention, au moins un additif usuel dans le domaine, tel que par exemple au moins un composé choisi parmi un agent émollient ou humectant, un agent gélifiant et/ou épaississant, un agent tensioactif, une huile, un agent actif, un colorant, un conservateur, un agent antioxydant, un agent actif, une poudre organique ou inorganique, un filtre solaire et un parfum.
- Un ou plusieurs agent(s) humectants, tels que les polyols (glycérine, diglycérine, le propylène glycol, le propanediol, le caprylyl glycol, le pentylène glycol, l'hexanediol), les sucres, les glycosaminoglycanes tels que l'acide hyaluronique et ses sels et esters ; et les polyquaterniums

Ledit agent humectant sera présent dans la composition à une teneur de l'ordre de 0.1 à 30%, de préférence 0.005 à 10% en poids total de la composition.
- Un ou plusieurs agent(s) émollient(s) qui peuvent être choisi(s) par exemple parmi les esters tels que les esters de jojoba, les esters d'acides gras et d'alcool gras (octyldodecyl myristate, triethylhexanoin, Dicaprylyl carbonate, Isostearyl isostearate, caprylic/capric triglycéride), les beurres tels que les beurres de karité (butyrospemum parkii butter extract, shea butter ethyl esters) ou de moringa (moringa oil/hydrogenated moringa oil esters), les cires (acacia decurrens flower wax & helianthus annuus cera seed seed wax, C10-18 triglycérides), les huiles végétales, le phytosqualane, les alcanes (undecane, tridecane)

Ledit agent émollient sera présent dans la composition à une teneur de l'ordre de 0.1 à 30%, de préférence 0.5 à 10% en poids total de la composition.
- Un ou plusieurs agent(s) gélifiants(s) et/ou épaississant(s) de la phase aqueuse, choisi par exemple parmi les dérivés cellulosiques, gommes d'origine végétale (guar, caroube, alginates, carraghenanes, pectines), d'origine microbienne (xanthane), les argiles (laponite), les homo- et copolymères réticulés ou non, hydrophiles ou amphiphiles, d'acide acryloylméthylpropane sulfonique (AMPS) et/ou d'acrylamide et/ou d'acide acrylique et/ou de sels ou d'esters d'acide acrylique (tel que l'Ammonium AMP/ VP Copolymer, l'Hydroxyethyl Acrylate/ AMPS Copolymer, le Sodium Acrylate/AMPS Copolymer, l'Acrylamide/AMPS Copolymer, l'Acrylates Copolymer, l'Acrylates/C10-30 Alkyl Acrylate Crosspolymer, le Polyacrylate crosspolymer-6, le sodium polyacrylate)

Ledit agent gélifiant et/ou épaississant sera présent dans la composition à une teneur de l'ordre de 0,1 à 10% en poids total de la composition.
- Un ou plusieurs agent(s) tensioactif(s), notamment :
   *les tensioactifs anioniques tels que les isethionates, les taurates, les sarcosinates, les glycinates, les glutamates, les phosphates tel que le C20-22 alkyl phosphate
   * les tensioactifs amphotères tel que les dérivés de la bétaine, les amphoacétates
   * les tensioactifs non ioniques tel que les dérivés de polyglycérol, les dérivés de glucosides (tel que le cetearyl glucoside, le c12-20 alkyl glucoside, l'arachidyl glucoside, le caprylyl/capryl glucoside, le decyl glucoside, le lauryl glucoside), les dérivés de xyloside, les lecithines.

Ledit agent tensioactif, sera présent dans une teneur de l'ordre de 0.1 à 15%, de préférence 0,5 à 10% en poids, par rapport au poids total de la composition ;
- Un ou plusieurs agent(s) filmogène(s) tel que les polymères d'origine naturelle, éventuellement modifiées, peuvent être choisis parmi la résine shellac, la gomme de sandaraque, la gomme arabique (ACACIA SENEGAL GUM), les dammars, les élémis, les copals, les polymères cellulosiques, les polymères extraits du fruit de Caesalpinia spinosa et/ou de l'algue Kappaphycus alvarezii, et leurs mélanges.

Ledit agent filmogène, sera présent dans une teneur de l'ordre de 0.1 à 15%, de préférence 0,5 à 10% en poids, par rapport au poids total de la composition.
- Un ou plusieurs agent(s) de coloration choisi(s) parmi les pigments, les nacres, les colorants solubles, de préférence solubles dans l'eau. Les pigments peuvent être blancs ou colorés, minéraux et/ou organiques. Les pigments minéraux peuvent être choisis parmi les oxydes de zirconium ou de cérium, ainsi que les oxydes de zinc, de fer, ou de chrome, le dioxyde de titane. Les nacres peuvent être choisies parmi les pigments nacrés, tels que le mica titane recouvert avec un oxyde de fer, le mica titane recouvert avec de l'oxychlorure de bismuth, le mica titane recouvert avec de l'oxyde de chrome, le mica titane recouvert avec un colorant organique, ainsi que les pigments nacrés à base d'oxychlorure de bismuth.

Ledit agent de coloration, sera présent dans une teneur de l'ordre de 0.01 à 20%, de préférence 2 à 15% en poids, par rapport au poids total de la composition.
- Un ou plusieurs agent(s) de charges choisi(s) parmi la lauroyl-lysine, l'amidon, le nitrure de bore et la silice

Ledit agent de charge, sera présent dans une teneur de l'ordre de 0.1 à 10%, de préférence 0,5 à 7% en poids, par rapport au poids total de la composition.
- Un ou plusieurs agent(s) actif(s) d'origine naturelle, biotechnologique ou synthétique ayant une activité biologique et ayant une efficacité sur la peau via des sites biologiques, par exemple choisi parmi les vitamines tels que la vitamine C et ses dérivés (ascorbyl glucoside, 3-o-ethyl ascorbic acid, le tétraisopalmitate d'ascorbyle) ,la vitamine A et ses dérivés, la vitamine E et ses dérivés, la vitamine B3 ou Niacinamide, le panthénol, les oligo éléments, l'allantoïne, l'adenosine, les peptides (Palmitoyl tetrapeptide-7, Palmitoyl Tripeptide-1, Palmitoyl Pentapeptide-4, Acetyl Dipeptide-1 Cetyl Ester, Acetyl Tetrapeptide-5), les extraits végétaux (glycyrrhiza glabra extract, centella asiatica leaf extract, secale cereale seed extract), les extraits de levures, les alpha hydroxyacides tels que l'acide glycolique ou lactique, l'acide tranexamique et ses dérivés tel que le cetyl tranexamique ester etc.

Ledit agent actif sera présent dans la composition à une teneur de l'ordre de 0.1 à 10% en poids total de la composition.

D'autres additifs habituellement utilisés en cosmétique peuvent également être présents dans la composition selon l'invention, notamment des conservateurs, des agents antioxydants ou des parfums bien connus dans le domaine technique.

L'homme du métier est en mesure de choisir, parmi l'ensemble de ces éventuels additifs, aussi bien la nature que la quantité de ceux qui seront ajoutés à la composition, de telle sorte que celle-ci conserve l'ensemble de ses propriétés.

L'invention est illustrée de manière non limitative par les exemples ci-dessous.

### Exemple 1 : Préparation d'un extrait alcoolique des parties aériennes de Plantago lanceolata, selon l'invention

Un extrait alcoolique des parties aériennes de *Plantago lanceolata.,* selon l'invention est préparé par un procédé comprenant les étapes suivantes :
a) le broyage de 1000g des parties aériennes séchées de *Plantago lanceolata,* en poudre
b) Extraction de la poudre dans 8300g d'éthanol 96° sous agitation à 60°C pendant 2h ;
   - Tamisage du mélange 1 obtenu à 50µm : le filtrat 1 est mis de côté et les drêches sont récupérées pour une nouvelle extraction ;
   - Extraction des drêches dans 5350g d'éthanol 96° sous agitation à 60°C pendant 2h
   - Tamisage du mélange 2 obtenu à 50µm : le filtrat 2 est récupéré et les drêches sont écartées ;
c) Le mélange formé par les filtrats 1 et 2 ; Filtration du surnageant à 4µm ;
   - Décoloration du filtrat obtenu pendant 3h à température ambiante sous agitation ; la décoloration s'effectue par ajout de 30% en masse de charbon actif par rapport à la masse sèche d'extrait ;
   - Filtration sur membranes à 4µm : élimination du charbon actif ;
d) Le filtrat ainsi obtenu est concentré par évaporation de l'éthanol pour obtenir un mélange concentré avec une matière sèche comprise en 10% et 11%
e) Ajout de 990 g de 1,3-propanediol pour obtenir un mélangé à 10% de matière sèche
   - Evaporation complète de l'éthanol à l'évaporateur à film raclé (concentration finale en éthanol inférieure à 0.5%).
   - Filtration finale à 4µm.

1075g d'extrait final sont obtenus.

### Exemple 2 : Modulation de l'expression de gènes impliqués dans la fonction barrière et l'inflammation dans des kératinocytes humains normaux traités avec l'extrait.

**Protocole :** Des kératinocytes épidermiques humains normaux issus de trois donneurs différents ont été ensemencés en plaque 24 puits et cultivés en milieu complémenté kératinocyte-SFM (k-SFM) pendant 48h à 37°C et 5% de CO2. Les cellules ont ensuite été incubées avec ou sans (condition non traitée) 0,03% d'extrait pendant 24h. Chaque condition a été effectuée en duplicat. Les ARN totaux ont été extraits à l'aide de TriPure Isolation Reagent^{®} selon le protocole préconisé par le fournisseur. Les ADN complémentaires ont été synthétisés et un transcriptome a été réalisé sur puce Affymetrix GeneChip Human Transcriptome Array 2.0. L'analyse bioinformatique des gènes dont l'expression est modulée a minima par un facteur 2 a été effectuée avec le logiciel Ingenuity Pathway Analysis (IPA^{®}, QIAGEN).

**Résultats** : Les voies biologiques significativement stimulées par l'extrait selon l'invention sont majoritairement en lien avec la fonction barrière cutanée. Il stimule notamment des marqueurs de la différenciation de l'épiderme tels que les SPRR, CNFN, LCE3D, SBSN, FLG1 ainsi que des marqueurs plus spécifiques des desmosomes tels que CDSN, DSG1 ou des jonctions serrées comme CLDN72. L'AQP9 est également un gène impliqué dans le maintien d'une bonne hydratation cutanée3. Les gènes impliqués dans le renforcement de la barrière physique cutanée ainsi que leur niveau de stimulation par l'extrait selon l'invention versus témoin non traité (niveau d'expression > 2) sont reportés dans le tableau I.

**Tableau I : Liste des gènes impliqués dans le renforcement de la fonction barrière dont l'expression est augmentée par l'extrait selon l'invention**

| **SSymbole** | **Nom du gène** | **Niveau d'expression (vs témoin non traité)** |
|---|---|---|
| SSPRR2D | small proline rich protein 2D | 22,850 |
| SSPRR2A | small proline rich protein 2A | 14,490 |
| SSPRR2B | small proline rich protein 2B | 13,820 |
| CCDSN | corneodesmosin | 7,520 |
| SSPRRIA | small proline rich protein 1A | 6,640 |
| CCNFN | cornifelin | 6,110 |
| SSPRR2G | small proline rich protein 2G | 5,070 |
| SSPRR2E | small proline rich protein 2E | 4,480 |
| SSPRRIB | small proline rich protein 1B | 3,700 |
| DDSG1 | desmoglein 1 | 3,260 |
| SSBSN | suprabasin | 3,240 |
| LLCE3D | late cornified envelope 3D | 3,200 |
| FFLG | filaggrin | 3,200 |
| SSPRR3 | small proline rich protein 3 | 2,990 |
| SSPRR2F | small proline rich protein 2F | 2,690 |
| AAQP9 | aquaporin 9 | 2,240 |
| SSPRR4 | small proline rich protein 4 | 2,130 |
| CCLDN7 | claudin 7 | 2,110 |

Par sa capacité à stimuler des cibles impliquées dans la différenciation de l'épiderme et la rétention d'eau dans la peau, l'extrait selon l'invention montre des propriétés cosmétiques de renforcement de la fonction barrière et d'hydratation.

D'autre part, comme indiqué dans le tableau II, l'extrait inhibe les voies biologiques de la neuro-inflammation et celle des interférons, des cytokines immunomodulatrices connues pour leur rôle régulateur dans l'inflammation cutanée4. Il diminue également l'expression de l'interleukine 33 (IL33) et de CXCL14, respectivement une cytokine et une chemokine participant également à l'inflammation de la peau.

**Tableau II : Liste des gènes impliqués dans les voies de l'inflammation dont l'expression est diminuée par l'extrait de Plantago.**

| Symbole | Nom du gène | Niveau d'expression (vs témoin non traité) |
|---|---|---|
| CCXCL14 | C-X-C motif chemokine ligand 14 | -11,300 |
| IIFI44L | interferon induced protein 44 like | -7,570 |
| IIFIT1 | interferon induced protein with tetratricopeptide repeats 1 | -3,990 |
| IIL33 | interleukin 33 | -3,640 |
| IIFI44 | interferon induced protein 44 | -3,500 |
| IIFIT3 | interferon induced protein with tetratricopeptide repeats 3 | -2,860 |

Par sa capacité à inhiber l'expression de différents gènes impliqués dans l'inflammation cutanée, l'extrait de Plantago présente un potentiel cosmétique apaisant.

### Exemple 3 : Stabilisation de mastocytes cutanés dans des explants, stressés ou non, traités avec l'extrait selon l'invention.

**Protocole** : Des explants de peau humaine ont été maintenus en survie en milieu adapté à 37°C et 5% de CO2. Vingt-quatre heures après mise en culture, ils ont été incubés avec ou sans (condition non traitée) 0,025% d'extrait pendant 24h puis ont été stressés avec le composé 48/80 déclenchant la dégranulation des mastocytes et traités à nouveau pendant 24h avec l'extrait. Chaque condition a été effectuée en triplicat. Les surnageants ont été collectés avant la stimulation avec le composé 48/80 pour doser le relargage basal d'histamine et 24h après pour observer l'effet de l'extrait après stimulation des mastocytes.

Résultats : Les mastocytes sont des cellules immunitaires sentinelles présentes dans les tissus en contact direct avec l'environnement tels que la peau. Ils contiennent des granules pleins de molécules pro-inflammatoire telles que l'histamine. Lorsque les mastocytes reçoivent des stimuli de stress par l'environnement, ils dégranulent, c'est-à-dire qu'ils libèrent le contenu de leurs granules, ce qui peut générer une sensation d'inconfort en surface de la peau, voire une inflammation7.

Comme le montre la Figure 1, l'extrait selon l'invention est capable de réduire le relargage d'histamine par les mastocytes en conditions basales comme stressées. Cela est dû au fait, comme indiqué en figure 2, que l'extrait est capable de diminuer le nombre de mastocytes ayant dégranulé.

En stabilisant les mastocytes qui relarguent ainsi moins d'histamine, l'extrait selon l'invention présente des propriétés cosmétiques apaisantes.

### Exemple 4 : compositions cosmétiques

Les compositions suivantes peuvent être préparées de façon classique pour l'homme du métier. Les quantités indiquées ci-dessous sont exprimées en pourcentages pondéraux. Les ingrédients en majuscules sont identifiés conformément à la dénomination INCI.

### A - émulsion huile/eau fluide

| **Nom INCI** | **(% W/W)** |
|---|---|
| Limnanthes alba (meadowfoam) seed oil | 1-10 |
| Camellia oleifera seed oil | 1-10 |
| Caprylic/capric triglycéride | 1-10 |
| Dicaprylyl carbonate | 1-5 |
| Squalane | 1-10 |
| Ammonium acryloyldimethyltaurate/VP copolymer | 0.1-5 |
| Hydroxyethyl acrylate/sodium acryloyldimethyl taurate copolymer & sorbitan isostearate & polysorbate 60 | 0.1-2 |
| Xanthan gum | 0.01-5 |
| Biosaccharide gum-1 | 0.01-5 |
| Sodium hyaluronate | 0.01-3 |
| Glycerin | 1-30 |
| Polyquaternium-51 | 1-10 |
| Tocopheryl acetate | 0.1-5 |
| Niacinamide | 0.1-5 |
| Polianthes tuberosa | 0.01-5 |
| Sodium pca | 0.5-5 |
| Saccharide isomerate | 0.5-5 |
| Extrait des parties aériennes de *Plantago lanceolata* selon l'invention | 0.001-10 |
| Yeast extract | 0.1-5 |
| CI 77891 (titanium dioxide) & aluminum hydroxide & magnésium chloride & sodium lauroyl glutamate & lysine | 1-10 |
| CI 77891 (titanium dioxide) & mica & tin oxide | 1-10 |
| Kappaphycus alvarezii Extract & Caesalpinia spinosa Fruit Extract | 0.1-5 |
| Titanium dioxide & stearic acid & alumina | 1-7 |
| Phenylbenzimidazole sulfonic acid | 1-3 |
| Glycols (Caprylyl Glycol and/or Pentylene Glycol and/or Butylène Glycol and/or propanediol) | 0,1-10 |
| Water | Qs 100 |

### b - émulsion huile/eau crème

| **Nom INCI** | **(% w/w)** |
|---|---|
| Jojoba esters | 1-5 |
| Limnanthes alba (meadowfoam) seed oil | 0.1-5 |
| Canola oil | 1-10 |
| Argania spinosa kernel oil | 0.1-10 |
| Moringa oil/hydrogenated moringa oil esters | 1-10 |
| C8-12 acid triglycéride | 1-5 |
| Lauroyl lysine | 1-5 |
| Camellia oleifera seed oil | 1-10 |
| Squalane | 1-10 |
| Ammonium acryloyldimethyltaurate/vp copolymer | 1-5 |
| Cetearyl alcohol & cetearyl glucoside | 1-7 |
| Sodium lauroyl glutamate | 0.1-1 |
| Hydrogenated lecithin | 0.1-5 |
| Chondrus crispus (carrageenan) | 0.1-5 |
| Sclerotium gum | 0,01-2 |
| Centella asiatica leaf extract | 0.1-5 |
| Adenosine | 0.1-0.5 |
| Niacinamide | 0.1-5 |
| Secale cereale (rye) seed extract | 0.1-5 |
| Palmitoyl tripeptide-1 & palmitoyl tetrapeptide-7 | 1-5 |
| Plankton extract | 0.1-5 |
| Yeast extract | 1-3 |
| Extrait des parties aériennes de *Plantago lanceolata* selon l'invention | 0.001-10 |
| Glycyrrhiza glabra extract | 0.001-5 |
| Tranexamic cetyl ester | 0.001-5 |
| Ascorbyl glucoside | 0.001-5 |
| Water | Qs 100 |

Ces compositions peuvent être appliquées tous les j ours, matin et/ou soir, sur la peau.

## Revendications

1. Extrait alcoolique des parties aériennes de *Plantago lanceolata,* comprenant au moins de l'aucubin, du catalpol et du verbascoside.

2. Extrait alcoolique des parties aériennes de *Plantago lanceolata* selon la revendication 1, comprenant :
- 10 à 50% en poids de sucres et de sorbitol, de préférence 20 à 40% en poids,
- 1 à 10% en poids d'aucubin, de préférence 6 à 9% en poids,
- 20 à 40% en poids de catalpol, de préférence 25 à 35% en poids, et
- 5 à 15% en poids de verbascoside, de préférence 10 à 12% en poids,
les pourcentages étant exprimés en poids, par rapport au poids total de l'extrait.

3. Extrait alcoolique des parties aériennes de *Plantago lanceolata* selon l'une quelconque des revendications précédentes, dans lequel les sucres comprennent du glucose et du sucrose, de préférence 1 à 7% en poids de glucose et 1 à 7% en poids de sucrose,
les pourcentages étant exprimés en poids, par rapport au poids total de l'extrait.

4. Procédé d'extraction des parties aériennes de Plantago lanceolata., comprenant les étapes suivantes :
a) le broyage des parties aériennes de Plantago lanceolata,
b) au moins une, de préférence au moins deux extractions desdites parties aériennes broyées en présence d'un premier solvant mono-alcoolique, chaque extraction étant suivie d'une étape de filtration ;
c) le mélange des filtrats liquides issus des extractions de l'étape b) ; et filtration du mélange obtenu et, optionnellement, décoloration du filtrat obtenu par adoption sur charbon actif ;
d) la concentration du mélange obtenu à l'étape c), pour obtenir un extrait ayant une teneur en matière sèche comprise entre 8% et 13%, le reste de l'extrait étant composé du premier solvant mono-alcoolique ;
e) l'ajout d'un deuxième solvant alcoolique à l'extrait concentré obtenu à l'étape d), ledit deuxième solvant alcoolique étant différent du premier solvant mono-alcoolique utilisé à l'étape b), suivi d'une élimination du premier solvant mono-alcoolique jusqu'à une concentration finale inférieure à 0.5% ; et optionnellement la filtration de l'extrait obtenu.

5. Procédé selon la revendication 4, **caractérisé en ce que** les parties aériennes de *Plantago lanceolata* mises en oeuvre à l'étape a) sont des parties aériennes séchées choisies parmi les fleurs, les feuilles, les tiges et leurs mélanges.

6. Procédé selon l'une quelconque des revendications 4 ou 5, **caractérisé en ce que** le premier solvant mono-alcoolique de l'étape b) est un monoalcool comprenant de 1 à 4 atomes de carbone, de préférence l'éthanol.

7. Procédé selon l'une quelconque des revendications 4 à 6, **caractérisé en ce que** les extractions de l'étape b) sont réalisées pendant une durée comprise entre 1 et 6 heures, de préférence entre 1 et 3 heures, à une température comprise entre 40°C et 80°C, de préférence entre 50 et 70°C.

8. Procédé selon l'une quelconque des revendications 4 à 7, **caractérisé en ce que** chaque extraction de l'étape b) est suivie d'une filtration sur un tamis de 50µm et/ou **en ce que** la filtration de l'extrait obtenu en c) mise en oeuvre à l'étape d) est effectuée sur un tamis ou une membrane de filtration de 4µm.

9. Procédé selon l'une quelconque des revendications 4 à 8, **caractérisé en ce que**, entre les étapes c) et d), est ajoutée une étape de décoloration du filtrat obtenu en c), de préférence par adsorption sur charbon actif, suivie d'une étape de filtration du filtrat décoloré obtenu, de préférence sur un tamis ou une membrane de filtration de 4µm.

10. Procédé selon l'une quelconque des revendications 4 à 9, **caractérisé en ce que** l'élimination de l'étape d) se fait par évaporation, puis l'ajout du deuxième solvant alcoolique est réalisé dans du 1,3-propanediol.

11. Extrait alcoolique de parties aériennes de *Plantago lanceolata,* **caractérisé en ce qu'**il est susceptible d'être obtenu par un procédé selon l'une quelconque des revendications 4 à 10.

12. Utilisation cosmétique d'un extrait alcoolique de parties aériennes de *Plantago lanceolata* selon l'une quelconque des revendications 1 à 3, comme actif apaisant.

13. Utilisation cosmétique d'un extrait alcoolique de parties aériennes de *Plantago lanceolata* selon l'une quelconque des revendications 1 à 3, pour améliorer l'hydratation et/ou améliorer la fonction barrière.

14. Composition cosmétique comprenant, dans un véhicule cosmétiquement acceptable, un extrait alcoolique de parties aériennes de *Plantago lanceolata* selon l'une quelconque des revendications 1 à 3.

15. Composition cosmétique selon la revendication 14 **caractérisée en ce qu'**elle est adaptée à une application par voie topique.
